# EUROPÄISCHE PATENTSCHRIFT

(11) **EP 3 160 953 B1**
(45) Veröffentlichungstag und Bekanntmachung des Hinweises auf die Patenterteilung: **07.10.2020**
(21) Anmeldenummer: 15734566.1
(22) Anmeldetag: 29.05.2015
(51) Int. Cl.: C07D 311/72

(54) **VERFAHREN ZUR GEWINNUNG VON VITAMIN E, STEROLEN UND/ODER TERPENEN AUS ÖLIGEN ODER FETTIGEN GEMISCHEN BIOLOGISCHER HERKUNFT**
PROCESS FOR OBTAINING VITAMIN E, STEROLS AND/OR TERPENES FROM OLEAGINOUS COMPOSITIONS OF BIOLOGICAL ORIGIN
PROCÉDÉ D'OBTENTION DE VITAMINE E, DE STÉROLS ET/OU DE TERPÈNES À PARTIR DE MÉLANGES HUILEUX OU GRAS D'ORIGINE BIOLOGIQUE

(30) Priorität: 24.06.2014 DE 102014009237
(43) Veröffentlichungstag der Anmeldung: 03.05.2017
(73) Patentinhaber: WeylChem Wiesbaden GmbH, 65203 Wiesbaden (DE)
(72) Erfinder: MORSCHHÄUSER, Roman, 55122 Mainz (DE); KCHIRID, Said, 63869 Heigenbrücken (DE); SCHOLZ, Hans Jürgen, 63755 Alzenau (DE)
(74) Vertreter: Ackermann, Joachim
(86) Internationale Anmeldenummer: PCT/EP2015/001094
(87) Internationale Veröffentlichungsnummer: WO 2015/197155

(56) Entgegenhaltungen:
- EP-A1- 0 610 742
- EP-A1- 1 122 250
- EP-A2- 2 448 905
- WO-A1-02/12222
- WO-A2-03/080778
- US-A- 5 190 618

## Beschreibung

Die vorliegende Erfindung betrifft ein Verfahren zur Gewinnung von Vitamin E, Sterolen und/oder Terpenen aus öligen oder fettigen Gemischen biologischer Herkunft sowie die Verwendung elektromagentischer Strahlung vorzugsweise im Mikrowellenband oder von Wärmetauschern zur Gewinnung solcher Inhaltsstoffe.

Die öligen oder fettigen Gemische entstammen Fetten und fette Ölen biologischer Herkunft, also pflanzlicher, tierischer oder mikrobieller Herkunft, und sind bekanntermaßen Gemische unterschiedlichster Inhaltsstoffe.

Bei Fetten und fetten Ölen handelt es sich um Stoffgemische, bei denen Ester des Glycerins mit drei, meist verschiedenen, überwiegend geradzahligen und unverzweigten aliphatischen Monocarbonsäuren, den Fettsäuren, den Hauptanteil ausmachen. Verbindungen dieser Art werden auch Triglyceride genannt. Neben den Glyceriden enthalten Fett und fette Öle Begleitsubstanzen, die üblicherweise in der Fachliteratur als "unverseifbare Anteile" zusammengefasst werden. Dabei handelt es sich um Inhaltsstoffe des sogenannten pflanzlichen Sekundärstoffwechsels, darunter Vitamin E, Sterole und/oder Terpene.

Je nachdem, ob das Stoffgemisch bei Raumtemperatur fest oder flüssig ist, spricht man von Fett oder fettem Öl. Bekannteste Fette sind die namensgebenden Stoffgemische aus verschiedenen Fettsäuretriglyceriden, die aus Tieren gewonnen werden. Der Begriff fettes Öl grenzt die (dünn)flüssigen Stoffgemische biologischer Herkunft von anderen Gruppen der Öle, beispielsweise der flüssigen Kohlenwasserstoffe, ab.

Fette und fette Öle aus biologischer Herkunft werden entweder aus tierischen Produkten, aus Pflanzen oder aus Mikroorganismen, wie Bakterien oder Hefen aber auch aus Algen oder Pilzen gewonnen. Tierische Fette können direkt aus Fettgewebe geschmolzen werden und fallen als Schmalz, Tran oder Talg an oder können aus Milch gewonnen werden. Die für Lebensmittel verwendeten pflanzlichen Öle und Fette können aus Ölpflanzen oder Ölsaat durch Pressung oder Extraktion mit Dampf, überkritischem CO₂ oder Lösungsmitteln gewonnen werden. Durch Raffination werden unerwünschte Inhaltsstoffe entfernt und die Fette oder fetten Ölen dadurch für die Nutzung veredelt.

Neuerdings werden erhebliche Mengen pflanzlicher Öle, insbesondere Rapsöl oder Palmöl, chemisch zu Biodiesel umgesetzt. Dazu werden die Öle einer Umesterung mit Methanol in Gegenwart meist basischer Katalysatoren unterworfen, wobei Fettsäuremethylester (FAME) und Glycerin gebildet werden. Erstere können direkt als Biodiesel eingesetzt oder herkömmlichem Kraftstoff beigemischt werden. Weiterhin können durch alkalische Hydrolyse aus Fetten oder fetten Ölen Seifen, die Alkalisalze von Fettsäuren, hergestellt werden. Dabei fällt ebenfalls Glycerin als Nebenkomponente an.

Die Verwendung von Fetten und fetten Ölen als Nahrungsmittel und in der Nahrungsmittelzubereitung sowie der Nahrungsmittelkonservierung ist weit verbreitet.

Native Fette und fette Öle sind direkt nach der Pressung oftmals für den direkten Verzehr nicht geeignet und müssen in einer Vorreinigung von Bitterstoffen, freien Fettsäuren, Farbstoffen und anderen unerwünschten Begleitstoffen, die den Geschmack und die Ästhetik beeinträchtigen, befreit werden. Dies wird mit zwei unterschiedlichen Techniken realisiert, die man als chemische- oder physkalische Raffination bezeichnet. Heutzutage wird aufgrund wirtschaftlicher Erwägungen im überwiegenden Maße die physikalische Aufreinigung zu Speiseölen bevorzugt. Dabei wird unter hohem Druck überspannter Wasserdampf bei Temperaturen oberhalb von 200°C durch das zu reinigende Öl geleitet. Dabei reißt der Wasserdampf alle darin flüchtigen Anteile mit sich, die sich nach der Kondensation und Entspannung des Druckes als sogeannte Desodorierungsdestillate gewinnen lassen. In der Literatur bezeichnet man diese "Abfallkomponenten" als Oleo-waste, als DDO ("Deodorization Distillates Oils"), als Kondensate ("Oil Physical Refining Condensate" oder "OPRC") oder im überwiegenden Falle im englischen Sprachraum als FAD ("Fatty Acid Distillates").

Unter dem Begriff "ölige und fettige Gemische" werden im Sinne der Erfindung Fette und fette Öle biologischer Herkunft als solche, aber auch die im Vorangegangenen benannten "Abfallkomponenten" , insbesondere DDO und OPRC, aber auch Abfallkomponenten von Fetten und Ölen, die in der Lebensmittelindustrie und Gastronomie anfallen, verstanden.

Die in den öligen und fettigen Gemischen enthaltenen "unverseifbare Anteile" sind Inhaltsstoffe des sogenannten Sekundärstoffwechsels, insbesondere des pflanzlichen Sekundärstoffwechsels. Diese chemisch und funktionell teilweise sehr unterschiedlichen Verbindungen werden im weiteren Verlauf dieser Beschreibung als "sekundäre Inhaltsstoffe" bezeichnet. Chemisch betrachtet sind diese sekundären Inhaltsstoffe komplizierte aliphatische, olefinische oder aromatische Alkoholkomponenten oder Terpene, die in Pflanzen aber auch Tieren die unterschiedlichsten physiologischen Wirkungen entfalten.

Ein nicht nur für die menschliche Physiologie sehr wertvoller Bestandteil dieser Substanzgruppe sind die Vitamine der E-Reihe. Die Grundstruktur aller Vitamin-E-Formen bildet ein an Position 6 hydroxylierter Chromanring, dessen Methylierung diese in eine α-, β-, γ- oder δ-Form unterteilt. Durch unterschiedlich gesättigte Seitenketten werden wieder zwei Hauptfamilien unterschieden, nämlich die gesättigen Tocopherole und die dreifach ungesättigten Tocotrienole. Auch weitere, eher als exotisch zu bezeichnende Spezies (Tocomonoenole), können im Vitamin E enthalten sein.

Vitamin E ist Bestandteil aller Membranen tierischer Zellen, wird jedoch nur von photosynthetisch aktiven Organismen wie Pflanzen und Cyanobakterien gebildet und muss daher von Tieren und Menschen über die Nahrung aufgenommen werden,

Von den acht wichtigsten Vertretern der natürlich vorkommenden Vitamin E Reihe ist das α- Tocopherol die Substanz mit der stärksten physiologischen Wirkung und daher auch mit der größten technische und wirtschaftliche Bedeutung. Die einzelnen Mitglieder der Tocopherolfamilie unterscheiden sich im Methylierungsgrad ihres Benzolkerns bzw. im Falle der Tocotrienole im Sättigungsgrad der Flügelgruppe.

Besonders hohe Gehalte an Vitamin E weisen pflanzliche Öle wie Weizenkeimöl (bis 2435 mg/kg Gesamttocopherol mit 70 % α-Tocopherol), Sonnenblumenöl (454-810 mg/kg Gesamttocopherol mit 86-99 % α-Tocopherol), rotes Palmöl (800 mg/kg Gesamt Vitamin E, davon 152 α-Tocopherol und 600 mg/kg Tocotrienole) und Olivenöl (46-224 mg/kg Gesamttocopherol mit 89-100 % α-Tocopherol) auf. Die dosis- und matrixabhängige Absorptionsrate liegt bei durchschnittlich 30 %.

Vitamin E wird auch synthetisch (u. a. von BASF, E. Merck (India) und DSM Nutritional Products) als ein racemisches Gemisch hergestellt. Synthetisches Tocopherol ist jedoch relativ instabil und wird daher meist noch mit einer Acetyl-Gruppe versehen (siehe auch dl-α-Tocopherylacetat). Dieses besitzt keine antioxidativen Eigenschaften. Es kann aber im Körper im Umfang von bis zu 50 % in natürliches Vitamin-E umgewandelt werden.

Ebenso wertvolle Bestandteile des unverseifbaren Anteils der öligen oder fettigen Gemische aus Fetten und fette Ölen biologischer Herkunft sind Sterole, die für die Pharma-, Kosmetik- und Nahrungsmittelindustrie wichtige biochemische Naturstoffe sind. Bei den Sterolen - auch Sterine genannt - handelt es sich um eine wichtige Untergruppe der Steroide. Grundgerüst ist das Sterin, ein Steran mit einer 3*β-*Hydroxygruppe. Nach ihrem Vorkommen lassen sich die Sterine unterteilen in Zoosterine (aus Tieren), Phytosterine (aus Pflanzen) und Mycosterine (aus Pilzen). Wichtige Zoosterine sind Cholesterin (Cholesterol) und Koprosterin, das im Darm durch Bakterien aus Cholesterin gebildet wird. Zu den Phytosterinen zählt das in Sojabohnen vorkommende Stigmasterin (Stigmasterol), das Camposterin (Camposterol) und auch das Sitosterin (Sitosterol). Auch in Weizenkeimlingen kommen mehrere Phytosterine vor. Zu der Gruppe der Mycosterine gehört z.B. das aus Hefen isolierbare Ergosterin (Ergosterol), das in enger Beziehung zu den Vitaminen der D-Reihe steht.

Eine weitere Gruppe von sekundären Inhaltsstoffen, die in Fetten und fetten Ölen biologischer Herkunft vorkommen und zu dem unverseifbaren Anteil gehören, sind die Terpene. Bei dieser, durch das erfindungsgemäße Verfahren abtrennbaren Substanzklasse, handelt es sich um eine stark heterogene und sehr große Gruppe von chemischen Verbindungen, welche in vielen Organismen natürlich vorkommen. Sie leiten sich formal vom Isopren ab und zeichnen sich dabei durch eine große Vielfalt an Kohlenstoffgerüsten mit funktionellen Gruppen aus. Die meisten Terpene sind pflanzlicher Herkunft und seltener tierischer Herkunft. In der Natur kommen überwiegend Kohlenwasserstoff-, Alkohol-, Glykosid-, Ether-, Aldehyd-, Keton-, Carbonsäure- und Ester-Terpene vor, aber auch Vertreter weiterer Stoffgruppen sind unter den Terpenen zu finden.

Terpene sind vielfach biologisch und pharmakologisch interessant. Sie können z.B. als umweltfreundliche Insektizide verwendet werden, indem sie als Pheromone Insekten in Fallen locken. Außerdem wirken viele antimikrobiell. Viele Terpene werden als Geruchs- oder Geschmacksstoffe in Parfümen und kosmetischen Produkten eingesetzt.

Unter Terpenen sind im Rahmen der vorliegenden Beschreibung neben Kohlenwasserstoffverbindungen sauerstoffhaltige Isoprenabkömmlinge zu verstehen, wobei letztere gelegentlich auch als Terpenoide bezeichnet werden.

Terpene werden in der Systematik der organischen Chemie zu den Lipiden gezählt. Die Zugehörigkeit zu den Terpenen begründet sich in einer gemeinsamen Biosynthese und der C₅-Regel, nicht in gemeinsamen Eigenschaften. Der gemeinsame Baustein aller Terpene ist das Isopren.

Generell wird zwischen acyclischen, mono-, bi-, tri-, tetra- und pentacyclischen Terpenen unterschieden, also Molekülen ohne, mit einem, mit zwei, drei, vier oder fünf Ringen. Weiterhin unterscheidet man die Terpene auch durch das Kohlenstoffgerüst, auf dem sie aufbauen. Außerdem werden sie über ihre sekundäre Stoffgruppenzugehörigkeit klassifiziert.

Die Terpene kann man in Isopreneinheiten unterteilen, welche die gleiche Anzahl von Kohlenstoffatomen haben. Terpene mit 5 Kohlenstoffatomen nennt man Hemiterpene (C₅), mit 10 Monoterpene (C₁₀), mit 15 Sesquiterpene (C₁₅), mit 20 Diterpene (C₂₀), mit 25 Sesterterpene (C₂₅), mit 30 Triterpene (C₃₀) und mit 40 Tetraterpene (C40). Terpene mit mehr als 8 Isopreneinheiten, also mit mehr als 40 Kohlenstoffatomen, nennt man Polyterpene (größer als C₄₀). Dabei wird die Isopreneinheit als halbes Terpen gezählt.

Ein besonders bevorzugtes Terpen ist das Squalen. Dabei handelt es sich um eine ungesättigte organische Verbindung mit der Summenformel C₃₀H₅₀ aus der Gruppe der Triterpene, die von allen höheren Organismen produziert wird. Der Stoff ist ein wesentlicher Bestandteil der Hautlipide und kommt ebenfalls im menschlichen Blutserum vor. Squalen kommt in verschiedenen Lebensmitteln in hohen Konzentrationen vor, beispielsweise in Ziegenmilch und in vielen pflanzlichen Ölen wie Olivenöl, Weizenkeimöl oder Reisöl. Das Hauptvorkommen sind Fischöle. Squalen wird industriell eingesetzt und zu Squalan hydriert, das als Salbengrundlage aber auch als Schmiermittel und Transformatorenöl Verwendung findet.

Bevorzugte Quellen dieser sekundären Inhaltsstoffe sind insbesondere Öle und Fette pflanzlicher Herkunft, insbesondere Acaiöl, Algenöl, Aprikosenkernöl, Arganöl, Avocadoöl, Babacuöl, Baumwollsamenöl, Behenöl, Borretschöl, Brennesselsamenöl, Cashew-Schalenöl, Cupuacu-Butter, Distelöl, Erdnussöl, Färberdistelöl, Hanföl, Hagebuttenkernöl, Haselnussöl, Jathrophöl, Jojobaöl, Kaffebohnenöl, Kakaobutter, Kamelienöl, Kokosöl, Kümmelöl, Kürbiskernöl, Leinöl, Leindotteröl, Macadamiöl, Maiskeimöl, Mandelöl, Mangobutter, Mohnöl, Nachtkerzenöl, Olivenöl, Palmöl, Palmkernöl, Papayasamenöl, Pekannussöl, Perillaöl, Pistazienöl, Rapsöl, Reisöl, Rizinusöl, Sanddornkernöl, Sanddornöl, Schwarzkümmelöl, Senföl, Sesamöl, Sheabutter, Sojaöl, Sonnenblumenöl, Traubenkernöl, Tungöl, Walnussöl, Wassermelonensamenöl oder Weizenkeimöl.

Je nach Herkunft enthalten pflanzliche fette Öle oder Fette einen Anteil von etwa 0,5 bis 5 Gew. %, tierische fette Öle oder Fette einen geringeeren Anteil an nicht verseifbaren sekundären Inhaltsstoffen, zu denen Vitamin E, Terpene und Sterole gezählt werden.

Desodorierungsdestillate (DDO oder OPRC) enthalten höhere Gehalte an sekundären Pflanzeninhaltsstoffen.

Zum Abtrennen dieser nicht-verseifbaren Bestandteile von Fettsäuren und Glyceriden (Fettkomponenten) stehen unterschiedliche Methoden zur Verfügung, wie in "The Encyclopedia of Vitamin E, ISBN 978-1-84593-075-2, S. 140-141 beschrieben. Eine übliche Methode zur Abtrennung der Fettkomponenten von Tocopherol ist die Veresterung der freien Fettsäuren gefolgt von einem Umesterungsschritt mit anschließender destillativer Abtrennung der Ester.

Stand der Technik ist, daß Fettsäuren und Glyceride durch Veresterung der Fettsäuren und durch Umesterung der Glyceride mit kurzkettigen Alkoholen, bevorzugt mit Methanol, in Fettsäuremethylester übergeführt und destillativ isoliert werden können. Eine Veresterung der Fettsäuren und Umesterung der Glyceride gelingt in herkömmlichen Verfahren nicht in einem einzigen Reaktionsschritt, sondern erfordert die Veresterung der Fettsäure in einem ersten Schritt und die Umesterung der Glyceride in einem nachfolgenden Schritt.

Die Umesterung der Glyceride erfolgt üblicher Weise im basischen Medium, da die Reaktionsgeschwindigkeit im sauren Millieu viel zu gering ist und deutlich drastischere Bedingungen erfordert. Die Anwesenheit von alkalischen Reagentien führt jedoch zur Neutralisation von freien Fettsäuren und stört die Umesterungsreaktion der Glyceride (partielle Verseifung). Aus diesem Grund wird in der Praxis eine saure Veresterung des freien Fettsäureanteils vorweggeschaltet, wie im folgenden Patent beschrieben.

US-A-5,190,618 lehrt, daß die nicht-verseifbaren Bestandteile von den Glyceriden und den freien Fettsäuren dadurch abgetrennt werden, indem im ersten Schritt die freien Fettsäuren mit einem kurzkettigen Alkohol, beispielsweise Methanol in Gegenwart eines sauren Katalysators, beispielsweise p-Toluolsulfonsäure, bei 65 bis 110°C verestert und in einem zweiten Reaktionsschritt die Glyceridanteile mit einem kurzkettigen Alkohol, beispielsweise Methanol, in Gegenwart eines basischen Katalysator, beispielsweise Natriummethanolat bei 30 bis 70°C umgeestert und die erhaltenen Fettsäurealkylester abdestilliert werden. Das im Rückstand angereicherte Tocopherol und Tocotrienol wird durch Kristallisation, lonenaustauschverfahren und Destillation in hohen Konzentrationen erhalten. Dieses Verfahren ist sehr aufwendig und hat zudem den Nachteil, daß Tocopherol sowie die ebenso vorhandenen Sterole unter den gewählten Reaktionsbedingungen im Sauren sehr leicht mit freien Fettsäuren verestert werden können. Im daher notwendigen alkalischen Reaktionsteil werden diese Ester anschließend wieder in die freien Komponenten gespalten, allerdings kommt es dabei zu einem sehr merklichen Abbau des Tocopherols aufgrund seiner oxidativen Instabilität im basichen Milieu.

US-A-5,487,817 beschreibt ein Verfahren zur Isolierung von Tocopherol und Sterolen aus einem Gemisch, bestehend im Wesentlichen aus Tocopherol, Sterolen, Fettsäuren und Glyceriden. In dem Verfahren werden die Sterole im Verlauf von 1 bis 12 Stunden bei 150 bis 250°C ohne saure oder basische Katalyse verestert und Fettsäurereste, Tocopherol und Sterolfettsäureester werden in mehreren Destillationsschritten bei 150 bis 220°C unter Vakuum getrennt. Die im Rückstand enthaltenen Sterolfettsäureester werden sauer-katalysiert umgeestert und vom Glycerid isoliert. Es ist bekannt (Acta Agric Scand 35: 136-138 (1985)), daß Temperaturen oberhalb von 120°C zu deutlichen Zersetzungen an Tocopherol führen, wodurch die Ausbeuten an Tocopherol verringert sind. Zudem wird eine geringere Menge an Tocopherol bei diesem Verfahren in Gegenwart von Fettsäuren ebenfalls verestert und bedingt einen weiteren Verlust an Vitamin E. Ebenso sind Sterole temperatur- und lichtempfindlich und werden unter den Bedingungen teilweise zerstört.

EP-A-2,448,905 beschreibt ein kontinuierliches Verfahren zur Herstellung von aliphatischen Carbonsäureestern, indem aliphatische Carbonsäuren mit Alkohol in Gegenwart eines Veresterungskatalysators unter Mikrowellenbestrahlung umgesetzt werden.

WO-A-2011/035853 beschreibt ein kontinuierliches Verfahren zur Herstellung von Fettsäuremethylestern durch Umesterung von Fettsäureestern mehrwertiger Alkohole, insbesondere Glyceride, mit Methanol in Gegenwart von basischen oder sauren Katalysatoren unter Mikrowellenbestrahlung. Das Verfahren ermöglicht in großtechnischen Prozessen, die Herstellung von hochreinen Fettsäuremethylestern aus nativen Fetten und Ölen, oder aus den Abfallprodukten aus deren Raffination, auch in Gegenwart von freien Säuren. Verfahren zur Isolierung weiterer Inhaltsstoffe aus den nativen Ölen oder Fetten oder aus den Abfallprodukten nativer Öle oder Fette ist nicht Gegenstand der Erfindung.

Die im Stand der Technik genannten Methoden zur Abtrennung von nicht-verseifbaren Inhaltsstoffen aus DDOs oder OPRCs sind energieintensiv und bezüglich der Ausbeute an den physiologisch besonders wertvollen sekundären Inhaltsstoffen, wie Vitamin E, insbesondere α-Tocopherol, Tocotrienolen, Sterolen und Terpenen, unbefriedigend.

Überraschend wurde gefunden, dass die sekundären Inhaltsstoffe Vitamin E, Sterole und Terpene aus öligen oder fettigen Gemischen von biologischer Herkunft in hohen Ausbeuten, vorzugsweise in Ausbeuten von größer als 90 %, bezogen auf die Ausgangskonzentration dieser sekundären Inhaltsstoffe, von den Fettkomponenten, bestehend im Wesentlichen aus Fettsäuren und Glyceriden abgetrennt werden können. Dieses erfolgt durch ein kontinuierliches Verfahren, bei dem in einer einstufigen sauer katalysierten Reaktion ein öliges oder fettiges Gemisch und einen einwertigen Alkohol enthaltendes Reaktionsgemisch in einem kontinuierlich arbeitenden Reaktor, welcher eine Heizzone aufweist, unter der Einwirkung hoher Temperaturen und hohen Druckes behandelt wird, wobei die Dauer des Energieeintrags und die Verweilzeit des Reaktionsgemisches in der Heizzone vergleichsweise kurz sind.

Das von den Komponenten Fettsäure, Fettsäureester und Glycerin abgetrennte Produkt enthält überraschenderweise hohe Ausbeuten an Vitamin E, Sterolen und/oder Terpenen.

Gegenstand der Erfindung ist ein kontinuierliches Verfahren zum Aufkonzentrieren oder Abtrennen von Vitamin E, Sterolen und/oder Terpenen, insbesondere Tocopherol, Tocotrienolen und/oder Sterolen aus öligen oder fettigen Gemischen biologischer Herkunft mit den Maßnahmen:
a) Vorlage eines Reaktionsgemisches enthaltend öliges oder fettiges Gemisch biologischer Herkunft, mindestens einen einwertigen Alkohol, bevorzugt Methanol und/oder Ethanol, und mindestens einen sauren Katalysator,
b) kontinuierliches Durchleiten des Reaktionsgemisches durch einen Reaktor, insbesondere durch einen Rohrreaktor, welcher eine Heizzone aufweist, in welcher das Reaktionsgemisch eine Temperaturerhöhung erfährt und beim Verlassen der Heizzone eine Temperatur zwischen 100°C und 190°C, bevorzugt zwischen 160°C und 180°C und besonders bevorzugt zwischen 165°C und 175°C, gemessen mittels Temperatursensor unmittelbar nach Verlassen der Heizzone, aufweist, und in dem das Reaktionsgemisch unter einem Druck zwischen 2 und 250 bar steht, so dass das Reaktionsgemisch in flüssigem, kritischem oder überkritischem Zustand vorliegt, vorzugsweise unter einem Druck zwischen 6 und 200 bar, ganz besonders bevorzugt zwischen 7 und 50 bar, und äußerst bevorzugt zwischen 15 und 25 bar,
c) Einstellen einer solchen Strömungsgeschwindigkeit des Reaktionsgemisches, dass dessen Verweilzeit in der Heizzone bis zu 10 Minuten beträgt, vorzugsweise bis zu 8 Minuten, besonders bevorzugt bis zu 5 Minuten, ganz besonders bevorzugt bis zu 1 Minute und äußerst bevorzugt bis zu 40 Sekunden,
d) Auftrennen des Produktgemisches aus Verfahrensschritt c) in eine polare Phase enthaltend das bei der Umesterung gebildete Glycerin, den nicht umgesetzten einwertigen Alkohol, den sauren Katalysator, das während der Veresterung des öligen oder fettigen Gemisches gebildete Reaktionswasser und in eine unpolare Phase enthaltend die durch Veresterung und durch Umesterung gebildeten Fettsäureester und darin gelöste und/oder dispergierte sekundäre Inhaltsstoffe, und
e) Abtrennen der durch Veresterung und durch Umesterung gebildeten Fettsäureester aus der unpolaren Phase aus Verfahrensschritt d) unter Erzeugung eines Rückstands, der Vitamin E, Sterole und/oder Terpene enthält.

Mit dem erfindungsgemäßen Verfahren werden sekundäre Inhaltsstoffe, ausgewählt aus Vitamin E, Sterolen und/oder Terpenen in hohen Konzentrationen ohne signifikante Zersetzungen, Umsetzungen, insbesondere ohne Veresterungen, oder Umlagerungen der chemischen Strukturen der Inhaltsstoffe gewonnen. Mit dem erfindungsgemäßen Verfahren werden im Verfahrensschritt c) Fettsäureester und freie Fettsäuren in ein und demselben Reaktionsgemisch verestert und umgeestert.

Der nach Verfahrensschritt e) des erfindungsgemäßes Verfahres erhaltene Rückstand enthält beispielsweise mindestens 70%, bevorzugt mindestens 80% und außerordentlich bevorzugt mindestens 95% der Ausgangsmenge anVitamin E, Sterolen und/oder Terpenen, insbesondere an α-Tocopherol und/oder an β-Sitosterol, Stigmasterol und Campestol, die nach bekannten Methoden, beispielsweise durch Extraktion, wie durch Extraktion mit überkritischem CO₂, oder Chromatographie isoliert werden können.

Eine Veresterung von Tocopherolen und Sterolen mit freien Fettsäuren hat in dem erfindungsgemäßen Verfahren wider Erwarten nicht oder kaum nachweisbar stattgefunden. Weder Tocopherolfettsäureester noch Sterolfettsäureester wurden mittels Gaschromatographie/ Massenspektroskopie detektiert. In dem nach Verfahrensschritt e) des erfindungsgemäßes Verfahres erhaltenen Rückstand wurden mittels GC/ MS freie Tocopherole und freie Sterole in hoher Reinheit, vorzugsweise in Konzentrationen von >95 %, bezogen auf die Gewichtsmenge des Ausgangsgehaltes an Tocopherolen und Sterolen ermittelt.

Mit dem erfindungsgemäßen Verfahren können die hitzeempfindlichen sekundären Pflanzeninhaltsstoffe Vitamin E, Sterole und/oder Terpene, insbesondere Tocopherole und Sterole in hohen Konzentrationen erhalten werden, ohne daß nennenswerte Umsetzungen oder Zersetzungen dieser sekundären Pflanzeninhaltsstoffe, beispielsweise Umsetzungen in Mengen von > 5%, bezogen auf die Gewichtsmenge dieser sekundären Pflanzeninhaltsstoffe im öligen oder fettigen Gemisch biologischer Herkunft, stattfindet.

Das erfindungsgemäße Verfahren zeichnet sich also dadurch aus, daß nur geringe Mengen an Vitamin E, Sterolen und Terpenen durch Zersetzung verloren gehen.

Das erfindungsgemäße Verfahren zeichnet sich insbesondere dadurch aus, daß Vitamin E und Sterole in freier, nicht veresterter Form auf der Basis von natürlichen Rohstoffen gewonnen werden können.

Die öligen oder fettigen Gemische aus fetten Öle oder Fetten biologischer Herkunft können als solche oder als Destillate einzeln oder in der Form von Mischungen im erfindungsgemäßen Verfahren eingesetzt werden oder kombiniert mit organischen Lösungs- oder Verdünnungsmitteln oder in der Form von Dispersionen. Beispiele für Lösungs- und Verdünnungmittel sind aliphatische oder aromatische Kohlenwasserstoffe oder aprotische organische Lösungsmittel. Die erfindungsgemäß bevorzugt verwendeten Destillate von Fetten und fetten Öle können allein, oder in Mischung zweier oder mehrerer solcher Destillate Verwendung finden.

Bevorzugt werden ölige oder fettige Gemische aus Ölen oder Fetten pflanzlichen Ursprungs eingesetzt, insbesondere Pflanzenöle, ausgewählt aus Acaiöl, Algenöl, Aprikosenkernöl, Arganöl, Avocadoöl, Babacuöl, Baumwollsamenöl, Behenöl, Borretschöl, Brennesselsamenöl, Cashew-Schalenöl, Cupuacu-Butter, Distelöl, Erdnussöl, Färberdistelöl, Hanföl, Hagebuttenkernöl, Haselnussöl, Jathrophöl, Jojobaöl, Kaffebohnenöl, Kakaobutter, Kamelienöl, Kokosöl, Kümmelöl, Kürbiskernöl, Leinöl, Leindotteröl, Macadamiöl, Maiskeimöl, Mandelöl, Mangobutter, Mohnöl, Nachtkerzenöl, Olivenöl, Palmöl, Palmkernöl, Papayasamenöl, Pekannussöl, Perillaöl, Pistazienöl, Rapsöl, Reisöl, Rizinusöl, Sanddornkernöl, Sanddornöl, Schwarzkümmelöl, Senföl, Sesamöl, Sheabutter, Sojaöl, Sonnenblumenöl, Traubenkernöl, Tungöl, Walnussöl, Wassermelonensamenöl oder Weizenkeimöl und davon ganz besonders bevorzugt Weizenkeimöl, Maiskeimöl, Rapsöl, Sojaöl, Sonnenblumenöl, Olivenöl und/oder Palmkernöl.

Besonders bevorzugt werden ölige oder fettige Gemische eingesetzt, die durch physikalische Raffination von Pflanzenölen erhaltenen werden und Desodorierungsdestillate ("Deodorization Distillates Oils" oder "DDO") bzw Kondensate ("Oil Physical Refining Condensate" oder "OPRC") genannt werden. Diese enthalten neben Fettsäuren und Glyceriden einen höheren Anteil an nicht-verseifbaren Bestandteilen, im Vergleich zum nicht raffinierten Öl.

Im erfindungsgemäßen Verfahren enhält das Reaktionsgemisch gemäß Verfahrensschritt a) einen oder mehrere einwertige Alkohole für die Veresterung von Fettsäuren oder die Umesterung von Triglyceriden und/oder Phospholipiden.

Dabei handelt es sich in der Regel um einen einwertigen aliphatischen Alkohol oder um ein Gemisch solcher Alkohole. Diese weisen im Allgemeinen ein bis sechs Kohlenstoffatome auf. Die einwertigen aliphatischen Alkohole können geradkettig oder verzweigt sein.

Beispiele für bevorzugt eingesetzte Alkohole sind Methanol, Ethanol, n-Propanol, isoPropanol, n-Butanol, iso-Butanol, tert.-Butanol, bevorzugt Methanol und Ethanol, besonders bevorzugt Methanol.

Das Molverhältnis von einwertigem aliphatischen Alkohol zu ver- oder umesterbaren Gruppen bestehend aus COOH-Gruppen und/oder aus Estergruppen ist größer 1.

In einer bevorzugten Ausführungsform des erfindungsgemäßen Verfahrens liegt das Molverhältnis von einwertigem aliphatischen Alkohol, insbesondere von Methanol, zu ver- oder umesterbaren Gruppen im Reaktionsgemisch bei 1 bis 20, vorzugsweise bei 1,5 bis 10, insbesondere bei 1,7 bis 6, und ganz besonders bevorzugt bei 2 bis 5.

Im erfindungsgemäßen Verfahren eingesetzte Ver- oder Umesterungskatalysatoren sind saure Katalysatoren oder deren Gemische. Dabei kann es sich um anorganische, metallorganische und/oder um organische saure Verbindungen handeln. Als saure anorganische Katalysatoren im Sinne der vorliegenden Erfindung sind Mineralsäuren einsetzbar, beispielsweise Salzsäure, Borsäure, Salpetersäure, Schwefelsäure, Phosphorsäure, Phosphonsäure oder hypophosphorige Säure; außerdem sind saure Salze einsetzbar, wie Aluminiumsulfathydrat, Alaun, saures Kieselgel oder saures Aluminiumhydroxid. Weitere saure anorganische Katalysatoren sind beispielsweise Aluminiumverbindungen der allgemeinen Formel Al(OR³)₃ und Titanate der allgemeinen Formel Ti(OR³)₄, wobei die Reste R³ jeweils gleich oder verschieden sein können und unabhängig voneinander gewählt sind aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, aus C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R³ in Al(OR³)₃ bzw. Ti(OR³)₄ jeweils gleich und gewählt aus Isopropyl, Butyl und 2-Ethylhexyl.

Bevorzugte saure metallorganische Katalysatoren sind gewählt aus Dialkylzinnoxiden (R³)₂SnO, wobei R³ wie oben stehend definiert ist. Ein besonders bevorzugter Vertreter für saure metallorganische Katalysatoren ist Di-n-butylzinnoxid, das als sogenanntes Oxo-Zinn oder als Fascat<(R)>-Marken kommerziell erhältlich ist.

Bevorzugte saure organische Katalysatoren sind organische Verbindungen, die saure Gruppen enthalten, beispielsweise Phosphatgruppen, Phosphonsäuregruppen, Sulfonsäuregruppen, Sulfatgruppen oder Carbonsäuregruppen. Besonders bevorzugte Sulfonsäuren enthalten mindestens eine Sulfonsäuregruppe und mindestens einen gesättigten oder ungesättigten, linearen, verzweigten und/oder cyclischen Kohlenwasserstoffrest mit 1 bis 40 C-Atomen und bevorzugt mit 1 bis 24 C-Atomen. Insbesondere bevorzugt sind aromatische Sulfonsäuren und speziell alkylaromatische Monosulfonsäuren mit einem oder mehreren C₁-C₂₈-Alkylresten und insbesondere solche mit C₁-C₂₂-Alkylresten.

Bevorzugte Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure, Dodecylbenzolsulfonsäure, Didodecylbenzolsulfonsäure, Naphthalinsulfonsäure.

Besonders bevorzugt für die Durchführung des erfindungsgemässen Verfahrens sind Borsäure, Phosphorsäure, Polyphosphorsäure und Polystyrolsulfonsäuren.

Insbesondere bevorzugt sind Titanate der allgemeinen Formel Ti(OR³)₄ und speziell Titantetrabutylat und Titantetraisopropylat.

In einer weiteren bevorzugten Ausführungsform werden im erfindungsgemäßen Verfahren saure, feste Katalysatoren eingesetzt. Beispiele dafür sind Zeolithe, Kieselgel, saure Schichtsilikate, wie Montmorillonit, und organische Ionenaustauscher.

Die sauren Katalysatoren werden typischerweise in Mengen von bis zu 10 Gew. %, bezogen auf die Gesamtmasse des Reaktionsgemisches verwendet, vorzugsweise in Mengen von 0,01 bis 10 Gew.-% und besonders bevorzugt von 0,02 bis 2 Gew.-%.

Das Reaktionsgemisch, enthaltend das ölige oder fettige Gemisch, sauren Katalysator und einwertigen Alkohol, wird durch einen Reaktor geleitet. Dabei erfährt das Reaktionsgemisch in einer Heizzone durch Zufuhr von Heizleistung eine starke Erwärmung. Dieses kann durch physischen Kontakt mit einer wärmeren Wand durch Wärmeübertragung erfolgen oder durch Wechselwirkung polarer oder ionischer Moleküle mit elektromagnetischen Feldern, beispielsweise mit Wellenlängen im Zentimeterbereich (Mikrowelle)

Erfindungsgemäß wird das Reaktionsgemisch in der Heizzone für eine Zeitspanne von bis zu 10 Minuten, vorzugsweise von bis zu 8 Minuten, besonders bevorzugt bis zu 5 Minuten, ganz besonders bevorzugt bis zu 1 Minute und äußerst bevorzugt bis zu 40 Sekunden, beispielsweise von 0,01 bis 40 Sekunden mit einer ausreichend hohen Heizleistung beaufschlagt. Dabei erfährt das Reaktionsgemisch eine starke Temperaturerhöhung und weist beim Verlassen der Heizzone eine Temperatur zwischen 100°C und 190°C, bevorzugt zwischen 160°C und 180°C und besonders bevorzugt zwischen 165°C und 175°C auf, gemessen mittels Temperatursensor unmittelbar nach Verlassen der Heizzone.

Das Reaktionsgemisch steht im Reaktor unter Druck zwischen 2 und 250 bar, so dass das Reaktionsgemisch in der Heizzone in flüssigem, kritischem oder überkritischem Zustand vorliegt, vorzugsweise unter einem Druck zwischen 6 und 200 bar, ganz besonders bevorzugt zwischen 7 und 50 bar, und äußerst bevorzugt zwischen 15 und 25 bar. Der flüssige, kritische oder überkritische Zustand des Reaktionsgemisches ist eine Voraussetzung für ein effizientes Aufheizen dieses Gemisches in der Heizzone, insbesondere für den Fall, dass dazu elektromagnetische Strahlung, wie beispielsweise Mikrowellenstrahlung eingesetzt wird.

Die Verweilzeit in der Heizzone wird durch die Auswahl einer geeigneten Stömungsgeschwindigkeit des Reaktionsgemisches durch diese Zone eingestellt. Eine weitere bevorzugte Möglichkeit im Sinne der Erfindung zur Anpassung der Verweilzit ist eine geeignete Auwahl der Apparategröße.

In einer Ausführungsform des erfindungsgemäßen Verfahrens schließt sich der Heizzone des Reaktors eine Verweilstrecke an. Das Reaktionsgemisch kann nach der Verweildauer in der Heizzone in dieser Verweilstrecke für eine Verweildauer von bis zu 30 Minuten, bevorzugt von 0,5 bis 600 Sekunden, besonders bevorzugt von 5 bis 300 Sekunden und ganz besonders bevorzugt von 30 bis 150 Sekunden, verbleiben.

Das Zuführen der erforderlichen Heizleistung in der Heizzone kann durch jede beliebige Vorrichtung erfolgen, welche in der Lage ist, kurzzeitig hohe Mengen an Heizleistung in das Reaktionsgemisch einzutragen. Beispiele für geeignete Vorrichtungen sind Wärmetauscher, insbesondere Rekuperatoren, oder elektomagentische Strahlung im Mikrowellenband

Als Rekuperatoren können die bekannten Typen eingesetzt werden. Beispiele dafür sind Plattenwärmeüberträger, Kapillarwärmetauscher, Mikroreaktoren, Spiralwärmeüberträger, Rohrbündelwärmeübertrager, U-Rohr-Wärmeübertrager, Mantelrohrwärmeüberträger, Heizregister oder Gegenstrom-Wärmeüberträger.

Bevorzugt wird das Reaktionsgemisch in der Heizzone mit hoher Heizleistung durch Erhitzen mit elektomagentische Strahlung im Mikrowellenband oder mit einem Wärmetauscher beaufschlagt.

Besonders bevorzugt ist die Heizzone in Form eines druckfesten, mikrowellen-transparenten Rohres ausgestaltet, das sich in einem geeignet dimensionierten Hohlraumresonator befindet, welcher in der Lage ist, ein resonantes elektromagnetisches Feld, vorzugsweise im Mikrowellenband, geeigneter Feldstärke zu erzeugen, mit dessen Hilfe das Reaktionsgut durch dielektrische Heizmechanismen erhitzt wird.

Die eingesetzte elektromagnetische Strahlung weist vorzugsweise eine Frequenz im Bereich von 300 MHz bis 30 GHz auf, insbesondere eine Frequenz von 915 MHz, 2,45 GHz oder 5,8 GHz.

Besonders bevorzugt wird der Hohlraumresonator im Monomode betrieben.

Durch die kurzzeitige Einwirkung von hohen Temperaturen und Drucken auf das Reaktionsgemisch finden in diesem sehr schnell und gleichzeitig Veresterungs- und Umesterungsreaktionen statt. Dabei wird der größte Teil der vorhandenen Triglyceride, Fettsäuren und Phospholipide in Fettsäureester umgewandelt und es wird Glycerin und Reaktionswasser freigesetzt.

In einer bevorzugten Ausführungsform der Erfindung erfolgt in Verfahrensschritt d) die Auftrennung des Produktgemisches aus Verfahrensschritt c) in eine polare Phase enthaltend das durch Umesterung gebildete Glycerin, den nicht umgesetzten einwertigen Alkohol, den sauren Katalysator und das Reaktionswasser und in eine unpolare Phase enthaltend die Fettsäureester und darin gelöste bzw. dispergiert die sekundären Inhaltsstoffe durch Phasenseparation, so daß die eine Phase im Wesentlichen Glycerin, einwertigen Alkohol, sauren Katalysator und Wasser enthält und die zweite Phase im Wesentlichen Fettsäureester und die darin gelösten bzw. dispergierten sekundären Inhaltsstoffe.

In einem nachfolgenden Schritt e) wird die Phase, enthaltend die sekundären Inhaltsstoffe von den Fettsäureestern abgetrennt, beispielsweise durch physikalische Trennverfahren, vorzugsweise durch Membrantrennung, durch chromatographische Verfahren, oder insbesondere durch Destillation, wobei der dabei anfallende Rückstand Vitamin E, Sterole und/oder Terpene enthält.

Ebenso geeignet sind Extraktionsverfahren, insbesondere mit überkritischem CO₂ zum Aufkonzentrieren oder Isolieren dieser Inhaltsstoffe.

Besonders bevorzugt werden die als "Molekulardestillationsverfahren" bezeichnete Dünnschichtverdampfung, Fallfilmverdampfung und Kurzwegverdampfung zur Aufkonzentration bzw. Abtrennung des Vitamins E, der Sterole und/oder der Terpene von den Fettsäuremethylestern eingesetzt.

Es können darüber hinaus Reste von unerwünschten Begleitkomponenten, insbesondere Reste nicht umgesetzter freier Fettsäure durch Wäsche mit Wasser gegebenenfalls auch unter Verwendung von Hilfsmitteln, wie Lauge oder Emulsionsbrechern, abgetrennt werden.

Der Vitamin E, Sterole und/oder Terpene enthaltende Rückstand aus Verfahrensschritt e) kann als solcher vermarktet werden oder dieser wird weiter aufgearbeitet, um einzelne gewünschte Inhaltsstoffe daraus zu isolieren. Dabei können die bekannten Trennverfahren zum Einsatz kommen.

Beispiele dafür sind Verfahren, bei denen die Auftrennung aufgrund der unterschiedlichen Siedepunkte der aufzutrennenden Verbindungen erfolgt. Beispiele für solche Verfahren sind Destillation, Rektifikation oder Strippen.

Weitere Beispiele dafür sind Verfahren, bei denen die Auftrennung aufgrund der unterschiedlichen Schmelzpunkte der aufzutrennenden Verbindungen erfolgt. Beispiele für solche Verfahren sind das Ausfrieren oder die fraktionierte Kristallisation.

Weitere Beispiele für Trennverfahren sind die Abtrennung von Substanzen durch Sublimation einschließlich der Gefriertrocknung.

Noch weitere Beispiele dafür sind Verfahren, bei denen die Auftrennung aufgrund der unterschiedlichen Löslichkeit der aufzutrennenden Verbindungen erfolgt. Beispiele für solche Verfahren sind chromatographische Verfahren oder extraktive Verfahren.

Bevorzugt werden Vitamin E, Sterole und/oder Terpene mit Hilfe des erfindungsgemäßen Verfahrens aufkonzentriert und isoliert. Dazu wird der Vitamin E, Sterole und/oder Terpene enthaltende Rückstand aus Schritt e) aufgearbeitet, um diese Wertstoffe daraus abzutrennen.

Insbesondere lässt sich das erfindungsgemäße Verfahren zum Aufkonzentrieren oder Isolieren von Sterolen und besonders von Vitamin E einsetzen.

Die Erfindung betrifft auch die Verwendung von Wärmetauschern, insbesondere von Rekuperatoren, oder von Mikrowellenapplikatoren in dem oben beschriebenen Verfahren zum Gewinnen von Vitamin E, Sterolen und/oder Terpenen aus öligen oder fettigen Gemischen biologischer Herkunft.

Die nachfolgenden Beispiele sollen die Erfindung näher erläutern ohne diese auf diese Beispiele einzuschränken.

Alle Prozentangaben sind als Gewichtsprozent zu verstehen.

### Beispiel 1: Vorschrift zur Gewinnung von Tocopherolkonzentraten aus Deodorizer Destillaten aus der Sojaöl- bzw. Sonnenblumenöl-Raffination

In einem beheizten 10 Liter Rührbehältnis wurden 3500 g eines DDO-Gemisches vorgelegt und unter Rühren mit 1450 g Methanol versetzt.

Das DDO-Gemisch besaß folgende Zusammensetzung: 50 Gew.-% DDO aus Sojaöl, 50 Gew-% DDO aus Sonnenblumenöl.

Nach erfolgter Mischung wurde die Reaktionsmischung mit 50g Schwefelsäure als Katalysator versetzt und die Mischung bei 50°C eintemperiert.

Zur Reaktionsführung wurde das Gemisch mit einer Geschwindigkeit von 6 Liter/h und einer Leistung von 0,8 kW durch eine kontinuierlich arbeitende- Mikrowellenapparatur (µ WaveFlow xx20 / 2450 MHz) der Firma Püschner GmbH gepumpt. Dabei wurde das Heizgut bei einem statischen Arbeitsdruck von 25 bar auf eine Temperatur von 170°C, gemessen mittels Pt100 Temperatursensor unmittelbar beim Verlassen der Bestrahlungszone, erhitzt.

Die Mikrowellenleistung wurde über die Versuchsdauer jeweils in der Art eingestellt, dass die gewünschte Temperatur des Reaktionsgutes am Ende der Bestrahlungszone konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen der Bestrahlungszone mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurückgespiegelte Mikrowellenenergie wurde mit Hilfe eines Prismensystems (Zirkulator) in eine Wasserlast geleitet. Aus der Differenz zwischen eingestrahlter Energie und Verlustleistung (ermittelt durch die Aufheizung der Wasserlast), wurde die in das Reaktionsgut eingetragene Mikrowellenenergie berechnet.

Mittels einer Hochdruckpumpe und eines geeigneten Druckentlastungsventils wurde die Reaktionsmischung in der Apparatur unter einen solchen Arbeitsdruck gesetzt, der ausreichte, um alle Edukte und Produkte bzw. Kondensationsprodukte stets im flüssigen Zustand zu halten. Die Reaktionsgemische wurden mit einer konstanten Flussrate durch die Vorrichtung gepumpt und die Verweilzeit in Bestrahlungszone und nachgeschalteter Verweilstrecke durch Modifizierung der Strömungsgeschwindigkeit eingestellt.

Die Verweilzeit in der Bestrahlungszone betrug ungefähr 35 Sekunden.

Die Verweilzeit in der nachgeschalteten Verweilstrecke betrug etwa 1 Minute.

Das Reaktionsprodukt wurde in einem Phasenabscheider aufgefangen und phasensepariert.

Der mittels ¹H-NMR bestimmte Umsatz (500 MHz in CDCl₃), bezogen auf die im Edukt Gemisch vorhandene freie Carbonsäure und Partialglyceride/Glyceride, betrug 93%.

Zur Entfernung von Methanol und Carbonsäureresten wurde die organische Oberphase (4350g) mit 1450g (1/3 der Menge) an 50°C warmer 3%iger Wasser/NaOH Lösung versetzt und 1 Stunde gerührt. Nach erneuter Phasenseparation wurde die wässrige Unterphase abgelassen und durch Anlegen eines Vakuums von 50mbar die organische Oberphase weitestgehend von Wasser und Methanol-Resten befreit. Es wurden 3670 g organische Oberphase erhalten.

Anschließend erfolgt eine destillative Abtrennung der flüchtigen Anteile in einem Dünnschichtverdampfer mit einer Verdampferfläche von 150 cm² und einem Vakuum von 2-3 mbar bei einer Vorlauftemperatur von 230°C.

**Tabelle 1: Destillationsbilanz:**

| Extrakt in g | Sumpf in g | Destillat in g | Kühlfallenrückstand in g | Rückstand in % | Destillat in % |
|---|---|---|---|---|---|
| 3670 | 510 | 3155 | 5 | 13,9 | 86,1 |

Alle nichtflüchtigen Anteile wurden am Boden des Dünnschichtverdampfers aufgefangen und als das gewünschte Konzentrat ausgetragen. Die Vitamin E Gehaltsbestimmung erfolgte miitels HPLC unter Verwendung entsprechender Tocopherol-Eichstandards.

Folgendermaßen wurde der Konzentrationsfaktor bestimmt:

Gemäß Destillaltionsbilanz wurden 86,1Gew-% der ursprünglichen organischen Oberphase destillativ abgetrennt und als Fettsäuremethylester mittels ¹H-NMR-Spektroskopie identifiziert.

Es verblieb ein Rückstand, bestehend aus 510 g Sumpf und 5 g Kühlfallenrückstand, wobei der Kühlfallenrückstand keinen sekundären Inhaltsstoff enthielt.

Die Tocopherolisomerengehalte, α-Tocopherol, β-Tocopherol, γ-Tocopherol und δ-Tocopherol, sowohl im eingesetzten Sojaöl und Sonnenblumenöl (DDO) als auch die Tocopherolisomerengehalte des Sumpfes wurden mittels Flüssigkeitschromatographie und Standard-Kallibrierung ermittelt und sind in Tabelle 2 zusammengefasst.

Der Gehalt an Tocopherol, bestehend aus den Tocopherolisomeren α-Tocopherol, β-Tocopherol, γ-Tocopherol und δ-Tocopherol im eingesetzten DDO aus 50 Gew.-% Sojaöl, 50 Gew-% Sonnenblumenöl betrug 1,91 Gew.-%. Unter der Annahme, daß nach der Umsetzung und Destillation 100% des Tocopherols aus den Tocopherolisomeren α-Tocopherol, β-Tocopherol, γ-Tocopherol und δ-Tocopherol sich im Sumpf befinden, ist ein Gewichtsanteil von 13,7 % an Tocopherol im Sumpf zu erwarten.

Analytisch bestimmt wurde ein Gehalt Tocopherol, bestehend aus den Tocopherolisomeren α-Tocopherol, β-Tocopherol, γ-Tocopherol und δ-Tocopherol von 13,1 Gew.-% bestimmt, was einer Ausbeute 94,9% an Tocopherol und einem Konzentrationsfaktor von 6,83 entsprach.

**Tabelle 2: Tocopherolgehalte im DDO, bestehend aus Soja- und Sonnenblumenöl, sowie Tocopherolgehalte nach erfolgter Umsetzung und Destillation gemäß Beispiel 1**

| | Tocopherol | | | | Summe |
|---|---|---|---|---|---|
| **DDO (Ursprung)** | α | **β** | Y | **δ** | |
| Soja | **0,15%** | **0,05%** | **0,82%** | **0,50%** | **1,52%** |
| Sonnenblume | **2,10%** | **0,13%** | **0,05%** | **0,02%** | **2,30%** |
| 50:50 Mischung | **1,13%** | **0,07%** | **0,44%** | **0,26%** | **1,91%** |

| **Konzentrat** | | | | | |
|---|---|---|---|---|---|
| theoretisch | **8,10%** | **0,50%** | **3,20%** | **1,9%** | **13,7%** |
| Bestimmt | **7,90%** | **0,60%** | **3,10%** | **1,5%** | **13,1%** |

### Beispiel 2:

Die Reaktionsführung erfolgte analog zum Besipiel 1 mit dem Unterschied, dass in diesem Falle ein reines Sonnenblumen DDO verwendet wurde.

Die Gehalte an Tocopherol vor der Umsetzung (roh) und nach Umsetzung und Aufarbeitung mit dem Dünnschichtverdampfer (2. und 3.TFE) sind in Tabelle 3 vermerkt.

Die Wiederfindungsrate wurde berechnet mit Hilfe der Destillationsbilanz. Sie liegt in diesem Bespiel bei 97%.

**Tabelle 3: Tocopherolgehalte im ursprünglichen unumgesetzten Sonnenblumenöl DDO, sowie Tocopherolgehalte nach erfolgter Umsetzung und Destillation gemäß Beispiel 2**

| **DDO (Ursprung)** | Tocopherol | | | | Wiederfin dungsrate |
|---|---|---|---|---|---|
| | **α** | **β** | **γ** | **δ** | |
| Sonnenblume | | | | | |
| **Ausgangsmaterial** | 1,7 | 0,1 | < 0,1 | < 0,2 | **n.b.** |
| **2.TFE** | 5,4 | 0,35 | < 0,1 | < 0,1 | **n.b.** |
| **3.TFE** | 7,50 | 0,48 | < 0,1 | < 0,2 | 97% |

### Beispiel 3:

Die Reaktionsführung erfolgte analog zum Beispiel 1 mit dem Unterschied, dass in diesem Falle ein Soja DDO verwendet wurde.

Die Gehalte an Tocopherol vor der Umsetzung (roh) und nach Umsetzung und Aufarbeitung mit dem Dünnschichtverdampfer (3a. und 3b.TFE) sind in Tabelle 4 vermerkt. Die Wiederfindungsrate wurde berechnet mit Hilfe der Destillationsbilanz. Sie liegt in diesem Bespiel bei 95% bzw 98% (Reproduktion)

**Tabelle 4: Tocopherolgehalte im DDO, bestehend aus Sojaöl, sowie Tocopherolgehalte nach erfolgter Umsetzung und Destillation gemäß Beispiel 3**

| **DDO (Ursprung)** | Tocopherol | | | | Wiederfin dungsrate |
|---|---|---|---|---|---|
| | **α** | **β** | **γ** | **δ** | |
| **Soja** | | | | | |
| **Ausgangsmaterial** | < 0,1 | < 0,1 | 0,81 | 0,58 | **n.b.** |
| **3a.TFE** | 0,87 | 0,24 | 4,50 | 3,20 | 0,98 |
| **3b.TFE** | 0,87 | 0,25 | 4,30 | 3,00 | 0,95 |

### Beispiel 4 und Vergleichsbeispiel 5: Mikrowellen assistierte Umsetzung eines Soja-basierten DDO bei unterschiedlichen Temperaturen

Die Reaktionsführung von Beispiel 4 und von Vergleichsbeispiel 5, Soja MW1 und Soja MW2, erfolgte analog zum Beispiel 1 mit dem Unterschied, dass bei Beispiel 4 mit einer Leistung von 0,75 kW und bei Vergleichsbeispiel 5 mit einer Leistung von 0,85 kW erhitzt wurde. Die jeweiligen Gemische in beidenVersuchen (Soja MW1 und Soja MW2) wurden bei einer Geschwindigkeit von 6 Liter/h gepumpt und bei Beispiel 4 mit einer Leistung von 0,75 kW und bei Vergleichsbeispiel 5 mit einer Leistung von 0,85 kW erhitzt. Dabei wurde das Heizgut bei einem statischen Arbeitsdruck von 25 bar auf eine Temperatur von 170°C (Beispiel 4) bzw 195°C (Vergleichsbeispiel 5) erhitzt.

Die Temperatur von 170°C und 195°C wurde mittels Pt100 Temperatursensor unmittelbar beim Verlassen der Bestrahlungszone gemessen.

Nach Abtrennung aller Begleitkomponenten wurde die jeweils entstandene organische Phase mittels HPLC unter Verwendung von entsprechenden Tocopherol-Eichstandards vermessen. Auf eine weitere Aufkonzentrierung durch Abtrennen des gebildeten Fettsäuremethylester (FAME) mittels Destillation wurde in beiden Fällen verzichtet.

**Tabelle 5: Gehalte an Tocopherol in einem Soja-basierten DDO vor und nach der Mikrowellen assistierten Umsetzung bei 170°C (Beispiel 4) und bei 195°C (Vergleichsbeispiel 5)**

| DDO (Ursprung) | Tocopherol | | | | Summe |
|---|---|---|---|---|---|
| | α | β | Y | δ | |
| Soja-DDO | 0,15 % | 0,05% | 0,82% | 0,50% | 1,52 % |
| Beispiel 4 (170°C) | 0,19 % | n.n | 0,77% | 0,51% | 1,47 % |
| Vergleichsbeispiel 5 (195°C) | n.n | n.n | 0,15% | < 0,1% | 0,15 % |

Tabelle 5 zeigt die ermittelten Werte im Vergleich zur Augangsmischung (Soja). Während bei 170°C im ersten Durchlauf eine 96%ige Wiederfindungsrate über alle Tocopherole ermittelt wurde, reduziert sich diese bei Umsetzung bei 195°C bei ansonsten gleichen Bedingungen auf 10%.

## Patentansprüche

1. Kontinuierliches Verfahren zum Aufkonzentrieren oder Abtrennen von Vitamin E, Sterolen und/oder Terpenen aus öligen oder fettigen Gemischen biologischer Herkunft mit den Maßnahmen:
a) Vorlage eines Reaktionsgemisches enthaltend öliges und/oder fettiges Gemisch biologischer Herkunft, mindestens einen einwertigen Alkohol und mindestens einen sauren Katalysator,
b) kontinuierliches Durchleiten des Reaktionsgemisches durch einen Reaktor, welcher eine Heizzone aufweist, in welcher das Reaktionsgemisch eine Temperaturerhöhung erfährt und beim Verlassen der Heizzone eine Temperatur zwischen 100°C und 190°C, gemessen mittels Temperatursensor unmittelbar nach Verlassen der Heizzone, aufweist, und in dem das Reaktionsgemisch unter einem Druck zwischen 2 und 250 bar steht, so dass das Reaktionsgemisch in flüssigem, kritischem oder überkritischem Zustand vorliegt,
c) Einstellen einer solchen Strömungsgeschwindigkeit des Reaktionsgemisches, dass dessen Verweilzeit in der Heizzone bis zu 10 Minuten beträgt,
d) Auftrennen des Produktgemisches aus Verfahrensschritt c) in eine polare Phase enthaltend das bei der Umesterung gebildete Glycerin, den nicht umgesetzten einwertigen Alkohol, den sauren Katalysator, das während der Veresterung des öligen oder fettigen Gemisches gebildete Reaktionswasser und in eine unpolare Phase enthaltend die durch Veresterung und durch Umesterung gebildeten Fettsäureester und darin gelöste und/oder dispergierte sekundäre Inhaltsstoffe, und
e) Abtrennen der durch Veresterung und durch Umesterung gebildeten Fettsäureester aus der unpolaren Phase aus Verfahrensschritt d) unter Erzeugung eines Rückstands, der Vitamin E, Sterole und/oder Terpene enthält.

2. Verfahren nach Anspruch 1, **dadurch gekennzeichnet, dass** das ölige oder fettige Gemisch pflanzlichen Ursprungs ist, bevorzugt Acaiöl, Algenöl, Aprikosenkernöl, Arganöl, Avocadoöl, Babacuöl, Baumwollsamenöl, Behenöl, Borretschöl, Brennessel-samenöl, Cashew-Schalenöl, Cupuacu-Butter, Distelöl, Erdnussöl, Färberdistelöl, Hanföl, Hagebuttenkernöl, Haselnussöl, Jathrophöl, Jojobaöl, Kaffebohnenöl, Kakaobutter, Kamelienöl, Kokosöl, Kümmelöl, Kürbiskernöl, Leinöl, Leindotteröl, Macadamiöl, Maiskeimöl, Mandelöl, Mangobutter, Mohnöl, Nachtkerzenöl, Olivenöl, Palmöl, Palmkernöl, Papayasamenöl, Pekannussöl, Perillaöl, Pistazienöl, Rapsöl, Reisöl, Rizinusöl, Sanddornkernöl, Sanddornöl, Schwarzkümmelöl, Senföl, Sesamöl, Sheabutter, Sojaöl, Sonnenblumenöl, Traubenkernöl, Tungöl, Walnussöl, Wassermelonensamenöl oder Weizenkeimöl.

3. Verfahren nach Anspruch 1, **dadurch gekennzeichnet, dass** das ölige oder fettige Gemisch ein DDO, ein OPRC oder ein FAD ist.

4. Verfahren nach einem der Ansprüche 1 bis 2, **dadurch gekennzeichnet, dass** dieses zum Abtrennen oder zum Aufkonzentrieren von Vitamin E, β-Sitosterol, Stigmasterol und/oder Campestol eingesetzt wird.

5. Verfahren nach mindestens einem der Ansprüche 1 bis 4, **dadurch gekennzeichnet, dass** es sich bei dem einwertigen Alkohol um einen einwertigen aliphatischen Alkohol mit ein bis sechs Kohlenstoffatome oder ein Gemisch davon handelt, insbesondere um Methanol und/oder Ethanol.

6. Verfahren nach mindestens einem der Ansprüche 1 bis 5, **dadurch gekennzeichnet, dass** es sich bei dem sauren Katalysator um eine anorganische, metallorganische und/oder organische saure Verbindung handelt,

7. Verfahren nach mindestens einem der Ansprüche 1 bis 6, **dadurch gekennzeichnet, dass** das Reaktionsgemisch in der Heizzone für eine Zeitspanne von bis zu 8 Minuten, vorzugsweise von bis zu 5 Minuten, besonders bevorzugt bis zu 1 Minuten, ganz besonders bevorzugt bis zu 40 Sekunden mit thermischer Energie beaufschlagt wird.

8. Verfahren nach mindestens einem der Ansprüche 1 bis 7, **dadurch gekennzeichnet, dass** das Reaktionsgemisch in der Heizzone eine Temperaturerhöhung erfährt und beim Verlassen der Heizzone eine Temperatur zwischen 160°C und 180°C und besonders bevorzugt zwischen 165°C und 175°C aufweist.

9. Verfahren nach mindestens einem der Ansprüche 1 bis 8, **dadurch gekennzeichnet, dass** das Reaktionsgemisch im Reaktor unter einem Druck zwischen 6 und 200 bar, ganz besonders bevorzugt zwischen 7 und 50 bar, und äußerst bevorzugt zwischen 15 und 25 bar, steht, so dass das Reaktionsgemisch in der Heizzone in flüssigem, kritischem oder überkritischem Zustand vorliegt.

10. Verfahren nach mindestens einem der Ansprüche 1 bis 9, **dadurch gekennzeichnet, dass** sich der Heizzone des Reaktors eine Verweilstrecke anschließt, in der das Reaktionsgemisch nach der Verweildauer in der Heizzone für eine Verweildauer von bis zu 30 Minuten, bevorzugt von 0,5 bis 600 Sekunden, besonders bevorzugt von 5 bis 300 Sekunden und ganz besonders bevorzugt von 30 bis 150 Sekunden, verbleibt

11. Verfahren nach mindestens einem der Ansprüche 1 bis 10, **dadurch gekennzeichnet, dass** das Zuführen von Heizleistung in die Heizzone durch Wärmetauscher, insbesondere durch Rekuperatoren, oder durch elektromagnetische Strahlung, insbesondere durch Mikrowellenstrahlung erfolgt.

12. Verfahren nach Anspruch 11, **dadurch gekennzeichnet, dass** die Heizzone in Form eines druckfesten, mikrowellen-transparenten Rohres ausgestaltet ist, das sich in einem geeignet dimensionierten Hohlraumresonator befindet, welcher in der Lage ist, ein resonantes elektromagnetisches Feld, vorzugsweise im Mikrowellenband, geeigneter Feldstärke zu erzeugen, mit dessen Hilfe das Reaktionsgut durch dielektrische Heizmechanismen erhitzt wird.

13. Verfahren nach Anspruch 12, **dadurch gekennzeichnet, dass** der Hohlraumresonator im Monomode betrieben wird.

14. Verfahren nach Anspruch 12, **dadurch gekennzeichnet, dass** die eingesetzte elektromagnetische Strahlung eine Frequenz im Bereich von 300 MHz bis 30 GHz aufweist, insbesondere eine Frequenz von 915 MHz, 2,45 GHz oder 5,8 GHz.

15. Verfahren nach mindestens einem der Ansprüche 1 bis 14, **dadurch gekennzeichnet, dass** das Abtrennen des sauren Katalysators, des durch Umesterung gebildeten Glycerins, des nicht umgesetzten einwertigen Alkohols, des Reaktionswassers und der durch Veresterung und durch Umesterung gebildeten Fettsäureester vom Reaktionsgemisch durch Phasentrennung, durch Membrantrennung, durch Extration oder insbesondere durch Destillation oder durch eine Kombination dieser Maßnahmen erfolgt.

16. Verfahren nach Anspruch 15, **dadurch gekennzeichnet, dass** das Abtrennen des sauren Katalysators, des durch Umesterung gebildeten Glycerins, des nicht umgesetzten einwertigen Alkohols, des Reaktionswassers und der durch Veresterung und durch Umesterung gebildeten Fettsäureester vom Reaktionsgemisch durch ein mehrstufiges Trennverfahren erfolgt.

17. Verfahren nach Anspruch 16, **dadurch gekennzeichnet, dass** das durch Umesterung gebildete Glycerin, das Reaktionswasser, der nicht umgesetzte Alkohol und der saure Katalysator in einer ersten Stufe durch Phasentrennung vom Reaktionsgemisch abgetrennt werden und in einer zweiten Stufe die durch Umesterung und Veresterung gebildeten Fettsäureester aus dem Reaktionsgemisch isoliert werden, wobei als Rückstand ein Gemisch verbleibt, das Vitamin E, Sterole und/oder Terpene enthält.

18. Verfahren nach mindestens einem der Ansprüche 1 bis 17, **dadurch gekennzeichnet, dass** der in Schritt e) erhaltene Vitamin E, Sterole und/oder Terpene enthaltende Rückstand weiter aufgearbeitet wird, um die gewünschten Inhaltsstoffe daraus zu isolieren, insbesondere durch Destillation, Rektifikation, Strippen, Ausfrieren, fraktionierte Kristallisation, Sublimation, Gefriertrocknung, chromatographische Verfahren oder extraktive Verfahren.

19. Verwendung von Wärmetauschern, insbesondere von Rekuperatoren, oder von Mikrowellenapplikatoren in einem Verfahren nach Anspruch 1 zum Gewinnen von Vitamin E, Sterolen und/oder Terpenen aus öligen oder fettigen Gemischen biologischer Herkunft

## Claims

1. A continuous process for concentrating or separating of vitamin E, sterols and/or terpenes from oily or fatty mixtures of biological origin comprising the measures:
a) providing a reaction mixture containing an oily and/or fatty mixture of biological origin, at least a monohydric alcohol and at least an acidic catalyst,
b) continuously conducting the reaction mixture through a reactor which has a heating zone in which the reaction mixture is heated and has when leaving the heating zone a temperature between 100 ° C and 190°C, measured with a temperature sensor immediately after leaving the heating zone, and in which the reaction mixture is under a pressure between 2 and 250 bar, that the reaction mixture is in liquid, critical or supercritical state,
c) adjusting the flow velocity of the reaction mixture that its dwell time in the heating zone is up to 10 minutes,
d) separating the product mixture from step c) into a polar phase containing the glycerol formed during the transesterification, the non-reacted monohydric alcohol, the acidic catalyst, and the reaction water that has been formed during the esterification of the oily or fatty mixture, and into a non-polar phase containing the fatty acid esters, that have been formed during the esterification and transesterification, and therein dissolved and/or dispersed secondary ingredients, and
e) separating the fatty acid esters formed by esterification and by transesterification from the non-polar phase from step d) under formation of a residue containing vitamin E, sterols and/or terpenes.

2. The process according to claim 1, wherein the oily or fatty mixture is of vegetable origin, preferably acai oil, algae oil, apricot kernel oil, argan oil, avocado oil, babacu oil, cotton seed oil, ben oil, borage oil, nettle seed oil, cashew shell oil, cupuaçu butter, thistle oil, peanut oil, safflower oil, hemp oil, rosehip seed oil, hazelnut oil, jathropha oil, jojoba oil, coffee bean oil, cocoa butter, camellia oil, coconut oil, cumin oil, pumpkin seed oil, linseed oil, cameline oil, macadamia oil, corn oil, almond oil, mango butter, poppy-seed oil, evening primrose oil, olive oil, palm oil, palm kernel oil, papaya seed oil, pecan oil, perilla oil, pistachio oil, rapeseed oil, rice oil, castor oil, sea buckthorn seed oil, sea buckthorn oil, black cumin oil, mustard oil, sesame oil, shea butter, soybean oil, sunflower oil, grape seed oil, tung oil, walnut oil, watermelon seed oil or wheat germ oil.

3. The process according to claim 1, wherein the oily or fatty mixture is a DDO, an OPRC or a FAD.

4. The process according to one of claims 1 or 2, wherein this is used for separation or concentration of vitamin E, β-sitosterol, stigmasterol and/or campestol.

5. The process according to at least one of claims 1 to 4, wherein the monohydric alcohol is an aliphatic monohydric alcohol with one to six carbon atoms or a mixture thereof, preferably methanol and/or ethanol.

6. The process according to at least one of claims 1 to 5, wherein the acidic catalyst is an acidic inorganic, metal-organic or organic compound.

7. The process according to at least one of claims 1 to 6, wherein the reaction mixture in the heating zone is treated with thermal energy for a time period of up to 8 minutes, preferably of up to 5 minutes, particularly preferred of up to 1 minute and most preferred of up to 40 seconds.

8. The process according to at least one of claims 1 to 7, wherein the reaction mixture experiences a temperature rise in the heating zone and has a temperature between 160°C and 180°C and particularly preferred between 165°C and 175°C when leaving the heating zone.

9. The process according to at least one of claims 1 to 8, wherein the reaction mixture in the reactor is under a pressure between 6 and 200 bar, particularly preferred between 7 and 50 bar, and most preferred between 15 and 25 bar, so that the reaction mixture in the heating zone is in liquid, critical or supercritical state.

10. The process according to at least one of claims 1 to 9, wherein a lingering line is connected to the heating zone of the reactor, wherein the reaction mixture after the dwell time in the heating zone remains for a retention period of up to 30 minutes, preferably from 0.5 to 600 seconds, particularly preferred from 5 to 300 seconds, and especially particularly preferred from 30 to 150 seconds.

11. The process according to at least one of claims 1 to 10, wherein the feeding of the required heating output in the heating zone is effected by heat exchangers, in particular recuperators, or by electromagnetic radiation, preferably by microwave radiation.

12. The process according to claim 11, wherein the heating zone is designed in the form of a pressure-resistant, microwave transparent pipe, which is located in a suitable sized cavity resonator, which is able to produce a resonant electromagnetic field, preferably in the micro wave band, of appropriate field strength, with whose help the reaction product is heated by dielectric heating mechanisms.

13. The process according to claim 12, wherein the cavity resonator is operated in the mono mode.

14. The process according to claim 12, wherein the used electromagnetic radiation has a frequency in the range from 300 MHz to 30 GHz, in particular a frequency of 915 MHz, 2.45 GHz or 5.8 GHz.

15. The process according to at least one of claims 1 to 14, wherein the separation of the acid catalyst, of the glycerol formed during the reaction, of the non-reacted monohydric alcohol, of the water formed during the reaction and of the fatty acid esters formed by esterification and transesterification from the reaction mixture is effected by phase separation, by membrane separation, by extraction or especially by distillation or by a combination of these measures.

16. The process according to claim 15, wherein the separation of the acid catalyst, of the glycerol formed during the reaction, of the non-reacted monohydric alcohol, of the water formed during the reaction and of the fatty acid esters formed by esterification and transesterification from the reaction mixture is effected by a multistage separation process.

17. The process according to claim 16, wherein the glycerol formed by transesterification, the water formed during the reaction, the non-reacted alcohol and the acid catalyst are separated from the reaction mixture in a first stage by phase separation and the fatty acid esters formed by esterification and by transesterification are isolated from the reaction mixture in a second stage, whereby in the sump a mixture comprising vitamin E, sterols and/or terpenes remains.

18. The process according to at least one of the claims 1 to 17, wherein the residue comprising vitamin E, sterols and/or terpenes from step e) is further worked-up to isolate the desired ingredients, preferably by distillation, by recification, by stripping, by freezing, by fractionated crystalization, by sublimation, by freeze drying, by chromatographic processes, or by extractive processes.

19. The use of heat exchangers, especially of recuperators, or of applicators for micro waves in a process according to claim 1 to obtain vitamin E, sterols and/or terpenes from oily or fatty mixtures of biological origin.

## Revendications

1. Procédé continu pour la concentration ou séparation de vitamine E, stérols et/ou terpènes à partir de mélanges huileux ou gras d'origine biologique comprenant les mesures suivantes :
a) le chargement d'un mélange réactionnel contenant un mélange huileux et/ou gras d'origine biologique, au moins un alcool monovalent et au moins un catalyseur acide,
b) le passage continu du mélange réactionnel au travers d'un réacteur, qui comprend une zone de chauffage, dans laquelle le mélange réactionnel subit une élévation de température et présente, à la sortie de la zone de chauffage, une température comprise entre 100 °C et 190 °C, mesurée au moyen d'un capteur de température directement après la sortie de la zone de chauffage, et dans lequel le mélange réactionnel se trouve sous une pression comprise entre 2 et 250 bar, de telle sorte que le mélange réactionnel se présente à l'état liquide, critique ou supercritique,
c) l'ajustement d'une vitesse d'écoulement du mélange réactionnel telle que son temps de séjour dans la zone de chauffage soit de jusqu'à 10 minutes,
d) la séparation du mélange de produits de l'étape de procédé c) en une phase polaire contenant la glycérine formée lors de la transestérification, l'alcool monovalent non réagi, le catalyseur acide, l'eau de réaction formée pendant l'estérification du mélange huileux ou gras et en une phase apolaire contenant les esters d'acides gras formés par estérification et par transestérification et des constituants secondaires dissous et/ou dispersés dans ceux-ci, et
e) la séparation des esters d'acides gras formés par estérification et par transestérification à partir de la phase apolaire de l'étape de procédé d) avec formation d'un résidu, qui contient de la vitamine E, des stérols et/ou des terpènes.

2. Procédé selon la revendication 1, **caractérisé en ce que** le mélange huileux ou gras est d'origine végétale, de préférence de l'huile d'açaï, de l'huile d'algue, de l'huile de noyau d'abricot, de l'huile d'argan, de l'huile d'avocat, de l'huile de babassu, de l'huile de coton, de l'huile de ben, de l'huile de bourrache, de l'huile d'ortie, de l'huile de noix de cajou, du beurre de cupuaçu, de l'huile de chardon, de l'huile d'arachide, de l'huile de carthame, de l'huile de chanvre, de l'huile de rose musquée, de l'huile de noisette, de l'huile de jatropha, de l'huile de jojoba, de l'huile de café, du beurre de cacao, de l'huile de camélia, de l'huile de coco, de l'huile de cumin, de l'huile de graine de courge, de l'huile de lin, de l'huile de lin bâtard, de l'huile de macadamia, de l'huile de maïs, de l'huile d'amande, du beurre de mange, de l'huile de pavot, de l'huile d'onagre, de l'huile d'olive, de l'huile de palme, de l'huile de palmiste, de l'huile de papaye, de l'huile de noix de pécan, de l'huile de périlla, de l'huile de pistache, de l'huile de colza, de l'huile de riz, de l'huile de ricin, de l'huile de graines d'argousier, de l'huile d'argousier, de l'huile de cumin noir, de l'huile de moutarde, de l'huile de sésame, du beurre de karité, de l'huile de soja, de l'huile de tournesol, de l'huile de pépin de raisin, de l'huile d'abrasin, de l'huile de noix, de l'huile de pépin de pastèque ou de l'huile de germes de blé.

3. Procédé selon la revendication 1, **caractérisé en ce que** le mélange huileux ou gras est un DDO, un OPRC ou un FAD.

4. Procédé selon l'une quelconque des revendications 1 à 2, **caractérisé en ce que** celui-ci est utilisé pour la séparation ou la concentration de vitamine E, de β-sitostérol, de stigmastérol et/ou de campestol.

5. Procédé selon au moins l'une quelconque des revendications 1 à 4, **caractérisé en ce que** l'alcool monovalent consiste en un alcool aliphatique monovalent contenant un à six atomes de carbone ou un mélange de celui-ci, notamment le méthanol et/ou l'éthanol.

6. Procédé selon au moins l'une quelconque des revendications 1 à 5, **caractérisé en ce que** le catalyseur acide consiste en un composé acide inorganique, métallo-organique et/ou organique.

7. Procédé selon au moins l'une quelconque des revendications 1 à 6, **caractérisé en ce que** le mélange réactionnel est exposé à de l'énergie thermique dans la zone de chauffage pendant une durée de jusqu'à 8 minutes, de préférence de jusqu'à 5 minutes, de manière particulièrement préférée de jusqu'à 1 minute, de manière tout particulièrement préférée de jusqu'à 40 secondes.

8. Procédé selon au moins l'une quelconque des revendications 1 à 7, **caractérisé en ce que** le mélange réactionnel subit une élévation de température dans la zone de chauffage et présente à la sortie de la zone de chauffage une température comprise entre 160 °C et 180 °C et de manière particulièrement préférée comprise entre 165 °C et 175 °C.

9. Procédé selon au moins l'une quelconque des revendications 1 à 8, **caractérisé en ce que** le mélange réactionnel dans le réacteur se trouve sous une pression comprise entre 6 et 200 bar, de manière tout particulièrement préférée entre 7 et 50 bar, et de manière préférée entre toutes entre 15 et 25 bar, de telle sorte que le mélange réactionnel se présente dans la zone de chauffage à l'état liquide, critique ou supercritique.

10. Procédé selon au moins l'une quelconque des revendications 1 à 9, **caractérisé en ce que** la zone de chauffage du réacteur est suivie par une section de séjour, dans laquelle le mélange réactionnel reste après la durée de séjour dans la zone de chauffage pendant une durée de séjour de jusqu'à 30 minutes, de préférence de 0,5 à 600 secondes, de manière particulièrement préférée de 5 à 300 secondes, et de manière tout particulièrement préférée de 30 à 150 secondes.

11. Procédé selon au moins l'une quelconque des revendications 1 à 10, **caractérisé en ce que** l'introduction de puissance de chauffage dans la zone de chauffage a lieu par des échangeurs de chaleur, notamment par des récupérateurs, ou par rayonnement électromagnétique, notamment par rayonnement micro-onde.

12. Procédé selon la revendication 11, **caractérisé en ce que** la zone de chauffage est configurée sous la forme d'un tube résistant à la pression, transparent aux micro-ondes, qui se trouve dans un résonateur à cavité dimensionné de manière appropriée, qui est apte à générer un champ électromagnétique résonant, de préférence dans la bande des micro-ondes, d'une puissance de champ appropriée, à l'aide duquel la matière réactionnelle est chauffée par des mécanismes de chauffage diélectriques.

13. Procédé selon la revendication 12, **caractérisé en ce que** le résonateur à cavité est exploité en monomode.

14. Procédé selon la revendication 12, **caractérisé en ce que** le rayonnement électromagnétique utilisé présente une fréquence dans la plage allant de 300 MHz à 30 GHz, notamment une fréquence de 915 MHz, 2,45 GHz ou 5,8 GHz.

15. Procédé selon au moins l'une quelconque des revendications 1 à 14, **caractérisé en ce que** la séparation du catalyseur acide, de la glycérine formée par transestérification, de l'alcool monovalent non réagi, de l'eau de réaction et des esters d'acides gras formés par estérification ou par transestérification du mélange réactionnel a lieu par séparation de phase, par séparation membranaire, par extraction ou notamment par distillation ou par une combinaison de ces mesures.

16. Procédé selon la revendication 15, **caractérisé en ce que** la séparation du catalyseur acide, de la glycérine formée par transestérification, de l'alcool monovalent non réagi, de l'eau de réaction et des esters d'acides gras formés par estérification et par transestérification du mélange réactionnel a lieu par un procédé de séparation à plusieurs étapes.

17. Procédé selon la revendication 16, **caractérisé en ce que** la glycérine formée par transestérification, l'eau de réaction, l'alcool non réagi et le catalyseur acide sont séparés du mélange réactionnel dans la première étape par séparation de phase et, dans une deuxième étape, les esters d'acides gras formés par transestérification et estérification sont isolés à partir du mélange réactionnel, un mélange restant en tant que résidu, qui contient de la vitamine E, des stérols et/ou des terpènes.

18. Procédé selon au moins l'une quelconque des revendications 1 à 17, **caractérisé en ce que** le résidu contenant de la vitamine E, des stérols et/ou des terpènes obtenu à l'étape e) est davantage traité, afin d'isoler les constituants souhaités, notamment par distillation, rectification, extraction, congélation, cristallisation fractionnée, sublimation, lyophilisation, des procédés chromatographiques ou des procédés extractifs.

19. Utilisation d'échangeurs de chaleur, notamment de récupérateurs, ou d'applicateurs de micro-ondes dans un procédé selon la revendication 1 pour l'obtention de vitamine E, de stérols et/ou de terpènes à partir de mélanges huileux ou gras d'origine biologique.
